# NEW EUROPEAN PATENT SPECIFICATION

(11) **EP 0 116 571 B2**
(45) Date of publication and mention of the opposition decision: **29.10.1997**
(45) Mention of the grant of the patent: 28.08.1991
(21) Application number: 83902407.2
(22) Date of filing: 01.07.1983
(51) Int. Cl.: A61K 39/395, A61K 39/00, A61K 35/14

(54) **METHOD FOR MAKING GAMMA GLOBULIN-CONTAINING COMPOSITIONS**
VERFAHREN ZUR HERSTELLUNG VON GAMMA-GLOBULIN ENTHALTENDEN ZUSAMMENSETZUNGEN
PROCEDE DE FABRICATION DE COMPOSITIONS CONTENANT DE LA GAMMA GLOBULINE

(30) Priority: 30.08.1982 US 413059
(43) Date of publication of application: 29.08.1984
(73) Proprietor: BAXTER INTERNATIONAL INC. (a Delaware corporation), Deerfield Illinois 60015 (US)
(72) Inventor: HOOPER, John, A., Santa Ana, CA 92706 (US); MANKARIOUS, Samia, Costa Mesa, CA 92626 (US); LIU-RASH, Catherine, R., Mission Viejo, CA 92691 (US)
(74) Representative: Weinhold, Peter, Dr.
(86) International application number: US8301016
(87) International publication number: WO8400891

(56) References cited:
- EP-A- 0 025 719
- EP-A- 0 035 616
- EP-A- 0 085 747
- US-A- 4 093 606
- US-A- 4 126 605
- US-A- 4 136 094
- US-A- 4 276 283
- US-A- 4 374 763
- US-A- 4 384 993
- US-A- 4 396 608
- BIOLOGICAL ABSTRACTS/RRM; ref.nr. 21008141 J.HOOPER et al.: "Purification and properties of normal human globulin G suitable for intra venous injection."
- VOX SANG. 29, 1975; pp. 101-123
- WO 84/00890

## Description

This invention relates to the ultrafiltration of gamma globulin-containing compositions. In particular, this invention is concerned with inhibiting the generation of anticomplement activity during the membrane ultrafiltration of gamma globulin concentrates.

Gamma globulin concentrates are compositions comprising immune globulin G and other proteins wherein the proportion of gamma globulin is greater than that found in normal pooled human plasma. The concentrates are well known and may be obtained by fractional precipitation of pooled plasma by alcohols and the like.

It is frequently desirable to ultrafilter such concentrates to remove salts, biologically active polypeptides and other undesired small molecules, as well as to concentrate protein in the solutions. Conducting ultrafiltration to remove small molecules without concommitantly increasing the concentration of protein is also known as dialysis. For the purposes herein, membrane ultrafiltration will be considered to include any procedure in which relatively small molecules in a solution or suspension are separated from comparatively large molecules by forcing or allowing the small molecules to pass through a membrane or barrier (such as a gel) having a pore size which is too small to allow the large molecules to pass through the membrane or barrier. Gamma globulin concentrates are generally ultrafiltered by the use of commercially available cassettes having membranes which pass molecules of less than about 100,000 Daltons. The concentrates can be dialyzed by exchanging dialysis fluids against bags constructed of the same membranes and holding the concentrates to be dialyzed.

Habeeb et al. ("Vox Sang." 32:143-158 [1977]) teach dialyzing a solution of Cohn Fraction II (a plasma fraction containing elevated gamma globulin levels obtained by ethanol precipitation), passing the solution through a DEAE (diethylaminoethyl)cellulose column to adsorb undesired proteins from the dialyzed solution, followed by recovering the enriched gamma globulin eluate. According to the authors, chromatography on DEAE-cellulose resulted in low levels of gamma globulin aggregates and anti-complement activity in the final immunoglobulin product.

Miekka and Gozze (Vox Sang. 29: 101-123 [1975]) who noticed an increase of anticomplement activity of IgG by pipetting during preparation of serial dilutions for the assay of this activity describe a stabilization of IgG by, for instance, albumin and polyethylene glycol, when added before the serial dilutions.

US-P 4 124 576 describes a new fractionation technique for the preparation of gamma globulin using polyethylene glycol as a precipitant , which according to the authors yields a gamma globulin substantially devoid of anticomplement activity and suitable for intraveneous administration.

It has been an objective of the art for quite some time to remove anticomplement activity. This activity has made it difficult to safely inject gamma globulin products intravenously since such injections frequently result in severe side effects. The alternative has been to inject the products intramuscularly, but this reduces the therapeutic efficacy of the gamma globulin.

In applicant's view, ultrafiltration and, to a lesser extent, chromatography of gamma globulin concentrates on anion exchange resins results in products having unacceptably high anticomplement activity. Accordingly, it is an object of this invention to employ ultrafiltration in manufacturing gamma globulin products, while also reducing or preventing the generation of anticomplementary activity in such products. This and other objects of the invention will become more apparent from consideration of this specification as a whole.

The anticomplement activity of gamma globulin products made by methods comprising ultrafiltration is considerably reduced by including one or more substantially non-surface-active stabilizers with gamma globulin concentrates during such ultrafiltration. Heretofore it has not been believed helpful or necessary to include stabilizers with gamma globulin during such steps, so it was particularly surprising to find that such stabilizers are necessary for the production of gamma globulin having acceptably low levels of anticomplement activity.

Therefore, the object of the present invention is a method for preparing gamma globulin having reduced anticomplement activity, comprising the steps of: a) Providing a gamma globulin concentrate, said concentrate including a polyether, said polyether being present in a concentration insufficient to precipitate proteins present in the concentrate and sufficient to reduce the anticomplement activity of the gamma globulin to be prepared, b) ultrafiltering said concentrate, and c) collecting said gamma globulin.

The starting gamma globulin concentrates are gamma globulin containing fractions obtained from plasma, tissue, recombinant microbial cultures or other sources. Suitable starting concentrates, if obtained from human plasma, will have a gamma globulin concentration per unit weight of protein that is higher than that found in normal pooled human plasma. Such concentrates generally contain greater than about 80%, preferably greater than about 96% gamma globulin by weight of total protein, the remaining protein including albumin, prothrombin complex and other plasma constituents. Cohn fractions such as fraction II are well known and suitable starting concentrates for ultrafiltration and ion exchange treatments. The concentrates are preferably essentially free of protease hydrolysis fragments of gamma globulin.

The concentrate may contain a high titer for a particular antigen or class of antigens of interest. This means that the concentrate will have a greater proportion of antibodies specific for such an antigen or class of antigens than is found in pooled normal plasma. Such "hyperimmune" globulin concentrates will usually contain high titers for various cellular or viral pathogens such as Clostridium or hepatitis.

Suitable stabilizers are hydrophilic macromolecules of the class of polyethers.

The macromolecule desirably can be metabolized completely or digested to innocuous fragments in the patient's circulation and/or excreted.

The polyethers will generally have an average molecular weight greater than 1000 Daltons, preferably about from 3000 to 50,000 Daltons. As a practical matter, polymers are infrequently available in which every molecule is of the same molecular weight. Accordingly, molecular weights disclosed herein shall be considered average, with the actual molecular sizes ranging plus or minus up to about 30%.

The macromolecule should be sufficiently water soluble to stabilize the concentrates. This means that the macromolecule is sufficiently hydrophilic to go into solution at a concentration of at least about 3% weight/volume in saline at room temperature. Obviously, this solubility requirement may vary depending upon the concentration of protein in the concentrate solution; lower protein concentrations will require less stabilizer, and thus the solubility can be lower. Saturated solutions may be used, and slight turbidity imparted by colloidal particles of excess water insoluble macromolecule is tolerable if the particles are not of a size to be a hazard to patients. However, it is preferred that the macromolecule be readily and completely water soluble at least in the amounts required for adequate stabilization.

This invention contemplates adding the macromolecule to gamma globulin concentrates as a solid or as a predissolved solution. It may be necessary to heat or otherwise treat the macromolecule to form a solution before it is combined with the concentrate. In such a case the macromolecule should not gel, precipitate or crystallize upon cooling or cessation of the dissolution treatment.

Generally polyethers provide for maximal gamma globulin stability.

Suitable polyethers are generally polyethers synthesized from hydroxylated monomers. Such polyethers are embraced within polymers having the following general structure: 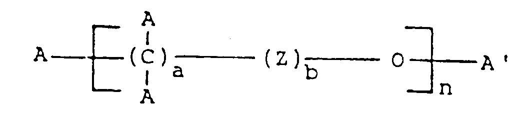 wherein A' is H, A is H, -OH, -NH₂, -CH₂OH, -CH₂NH₂, or -CH₂COOH, a is 1 to 3, n is greater than about 20, Z is -CH₂-, b is zero or 1; and block copolymers of such polymers. Polyethylene glycol is the preferred polymer.

Preferably the polyethers are substantially free of charged groups.

The amount of polyethers to be used is subject to some discretion. The optimal quantity should be determined by routine experimentation, but it will generally range in a weight ratio to the total protein present in the gamma globulin concentrate of about from 0.0075 to 0.062, preferably about from 0.01 to 0.05. This quantity should be more than the trace or residual levels remaining after a precipitation step in which gamma globulin is precipitated from a solution containing the macromolecule. Thus, even if the macromolecule is present in solutions from which gamma globulin is precipitated as part of an earlier purification procedure, a supplementary amount of the macromolecule should be added to the redissolved, precipitated protein before it is ultrafiltered.

The polyether may be used in concert with other groups of gamma globulin stabilizers, low molecular weight polyols and amino acids. The use of such stabilizer along with the polyethers permits the use of less of each stabilizer than would have been the case if any one stabilizer had been employed alone.

The polyol is a compound having a molecular weight of less than 1000 Daltons and a high degree of substitution by hydroxyl groups, generally up to about seven hydroxyl groups per molecule. The preferred polyols are sugar alcohols or reducing and nonreducing mono, di and trisaccharides. Exemplary polyols include mannitol, sorbitol, glucose, mannose, lactose, fructose and maltose. Glucose is most preferred. In case an amino acid is added this is generally an aliphatic or neutral amino acid, i.e. leucine, isoleucine, valine or glycine. Glycine is preferred.

The amount of polyol to be used shall be determined in the same fashion as described above for the macromolecule, although typical amounts for polyol range in a weight ratio to total protein in the concentrate from about 0.05 to 1.25, preferably 0.1 to 0.5.

Preferably the stabilizer is free of hydroxyethyl starch.

Ultrafiltration of gamma globulin concentrates may precede or follow ion exchange treatment. When ultrafiltration precedes the ion exchange step, it is generally conducted as dialysis for purposes of removing undesired molecules, generally of small molecular weight such as salts or solvents. Ultrafiltration after ion exchange treatment is frequently to increase the protein concentration prior to lyophilization or for storage. Gamma globulin stabilizers are useful in either situation, or in ultrafiltration and ion exchange adsorption alone.

The concentration of total protein in concentrates for dialysis ultrafiltration is generally about from 30 to 60 g/L, of which about 90% by weight is gamma globulin. The solution to be ultrafiltered is placed in a dialysis membrane bag, such membranes having the capacity to pass molecules of less than about 100,000 Daltons. The bag is then immersed in a circulating dialysis fluid. Small molecules will diffuse into the dialysis fluid, which is customarily drawn off and substituted with fresh fluid as the dialysis progresses. The dialysis is stopped when the desired concentration of small molecule is reached in the dialysis retentate. This is a function of the identity of the small molecules as well as what fractionation steps, if any, are to follow the dialysis. Thus the dialysis time, temperature and other parameters will be optimized in each case as required.

Ultrafiltration concentration is used to increase protein (and thus gamma globulin) concentration. It can also be used in a dialysis mode by adding dialysis fluid to the concentrate solution and continuing to ultrafilter until a higher, desired protein concentration is reached, usually about from 4 to 8 percent protein weight/volume, during which the undesired small molecules also are removed.

Ultrafiltration dialysis or concentration is accomplished on a large scale by the use of commercially available ultrafiltration membrane systems. These are devices in which the geometry of the ultrafiltration system has been optimized for the most efficient removal of water and/or small solute molecules from protein solutions, including directing the retentate flow laterally across the membrane surface to reduce clogging of the filter membrane pores by retained proteins.

Generally dialysis of the solution of gamma globulin concentrate is against at least four volumes of a buffered dialysing solution.

In accordance with this invention the solution of gamma globulin concentrate will contain one or more stabilizers in the amounts described above. If the ultrafiltration membrane has a molecular weight cut-off higher than that of the stabilizer then preferably any dialysis solution will contain a similar concentration of stabilizer as to prevent a reduction in stabilizer concentration during treatment.

Methods for purifying gamma globulin concentrates by the use of ion exchange resins are known. For example, see Reif, "Immunochemistry" 6:723-731 (1969), Hoppe et al., "Vox Sang." 25:308-316 (1975) and U.S. Patent 4,312,949. Ion exchange resins are water insoluble materials substituted with positively or negatively charged ionic groups, or mixtures of such groups (amphoteric resins). They are generally granular and have been equilibrated before use with a physiologically acceptable ion such as chloride, hydroxyl or alkali metal ions. The resins are selected to adsorb desired or undesired proteins from admixture with the target protein. Selection and use of appropriate resins is within the skill of the ordinary artisan. Generally, an anion exchange resin such as DEAE-cellulose is used in the purification of globulin concentrates because it is capable of preferentially adsorbing prothrombin complex proteins from the concentrate.

The solution of gamma globulin concentrate is recovered by centrifuging or filtering to remove the insoluble resin and its bound protein. The ion exchange adsorption process can be repeated several times. In accordance with this invention, the solution of gamma globulin concentrate which is incubated with the ion exchange resin will contain one or more stabilizers in the amounts described above.

It is possible to perform an ultrafiltration step simultaneously with the ion exchange procedure. This is readily accomplished by simply mixing an ion exchange resin with the concentrate solution and ultrafiltering the resulting mixture. Generally, however, it is preferred that the ultrafiltration and ion exchange treatments be sequential, with ultrafiltration dialysis preceding ion exchange treatment.

It is not necessary to separate the stabilizers from the gamma globulin concentrate following ultrafiltration and ion exchange treatments so long as the stabilizers are physiologically acceptable in the amounts employed. Thus, in the broad preferred embodiment, a solution of Cohn Fraction II or other concentrate is dissolved in a solution having a pH of about from 5.0 to 5.6, usually 5.3, which contains polyethylene glycol at a concentration ranging from about 0.05% (w/v) to just below that at which proteins in the concentrate will precipitate, generally about 5-8% (w/v).
Preferably the polyethylene glycol is present in a concentration of from 0.1% to 2.0% (w/v) of concentrate solution. This is dialyzed against about 3 to 5 volumes of a solution containing about half as much polyethylene glycol (w/v) as is present in the concentrate solution. The dialysis retentate is recovered and the pH of the concentrate adjusted to about 8.0 to optimize the ion exchange adsorption to follow. Hydrated DEAE Sephadex is mixed with the concentrate solution to adsorb undesired protein and the DEAE Sephadex then separated from the concentrate. A polyol, amino acid and albumin are added in amounts sufficient to inhibit the generation of anticomplement activity during lyophilization, the concentrate sterile filtered, filled into containers, lyophilized and the containers hermetically sealed. This product contains less than 200 Ch50 units of anticomplementary activity/gram of protein. Without the use of polyethylene glycol during the dialysis and ion exchange adsorption steps the anticomplementary activity/gram of protein is frequently over 10 times higher.

### EXAMPLE

Dialysis was performed in a Millipore dialysis system which consisted of two Millipore Pellicon® cassette holders (stainless steel), twenty Millipore PTHK cassettes (100,000 Dalton cut-off), and two diaphragm pumps. Prior to dialysis, the system was washed to remove bacterial pyrogens. After depyrogenation, the system was washed with several liters of cold dialysis buffer. The dialysis procedure was performed at 5°C. Dialysis flow rates and pump speeds were adjusted to avoid the generation of foam. All solutions were prepared using pyrogen-free water.

An aqueous starting solution of Cohn Fraction II at a protein concentration of 50g/liter and having a pH of 5.3 was dialyzed against 4 volumes (approximately 300 liters) of a solution which contained 20 mM NaCl and 0.5 g/L PEG-4000. The Fraction II solution was pumped through the Millipore cassettes where materials with a molecular weight of less than 100,000 Daltons were removed by ultrafiltration. The retentate, which contained concentrated immune globulins (150,000 Daltons and up), was returned to and mixed with the immune globulin solution undergoing dialysis. Simultaneously, the above described dialysis solution was continuously pumped into the immune globulin solution at the same rate as filtrate was produced and removed via the cassettes.

The immune globulin solution was stirred constantly to distribute the retentate and the continuously added dialysis solution. By adjusting the pump supplying the dialysis solution and the pump feeding the mixed retentate and dialysis solution into the cassettes, the volume and protein concentration of the immune globulin solution remained constant. When the 300 liters of dialysis solution was depleted, the immune globulin solution was concentrated to a protein concentration of 55 mg/ml using the cassettes in conventional fashion.

The concentrated immune globulin solution was treated with an ion exchange resin in the following fashion. Tris base was added to the solution to produce a final tris concentration of 0.025M and the pH was adjusted to 8.0 ± 0.1. Hydrated DEAE (diethylaminoethyl) Sephadex A-50 was mixed with the solution at a concentration of 5 g/g of protein and the mixture was agitated for 3 hours at 5°C, whereafter the Sephadex A-50 was filtered from the solution. NaCl, dextrose, glycine and albumin were added to the solution to make final concentrations, respectively of 0.85%, 2.0%, 2.25% and 0.1%, pH was adjusted to 7.0 and the protein concentration adjusted to 5.2%. This solution contained about 1.3 mg of PEG-4000/ml, carried over from the dialysis step. The solution was sterile filtered, filled into vials, lyophilized and the vials sealed.

## Claims

1. Method for preparing gamma globulin having reduced anticomplement activity, comprising the steps of:
a) Providing a gamma globulin concentrate, said concentrate including a polyether, said polyether being present in a concentration insufficient to precipitate proteins present in the concentrate and sufficient to reduce the anticomplement activity of the gamma globulin to be prepared,
b) ultrafiltering said concentrate, and
c) collecting said gamma globulin.

2. The method of claim 1 further comprising, after step b), the step of treating said concentrate with an anion exchange resin.

3. The method of claim 1 wherein the polyether is substantially free of charged groups.

4. The method of claim 1 wherein the polyether has a molecular weight greater than 1000 Daltons.

5. The method of claim 1 wherein said polyether is polyethylene glycol.

6. The method of claim 5 wherein said polyethylene glycol has an average molecular weight of 4000 Daltons.

7. The method of claim 5 or 6 wherein the polyethylene glycol is present in a concentration of from 0.05% to 2.0% by weight/volume of concentrate solution.

8. The method of claim 1 wherein the ultrafiltration is conducted at a pH of from 5.0 to 5.6.

9. The method of claim 1 wherein the ultrafiltering comprises dialysing said gamma globulin concentrate against at least four volumes of a dialyzing solution.

10. The method of claim 9 wherein the dialyzing solution also contains a polyether.

11. The method of claim 1 further comprising filtering said gamma globulin to retain cellular microorganisms, filling said gamma globulin into containers, lyophilizing said gamma globulin, and hermetically sealing the containers.

12. The method of claim 1 wherein said gamma globulin concentrate comprises an alcohol precipitate of gamma globulin-containing protein from human plasma.

13. The method of claim 1 wherein said gamma globulin concentrate contains greater than 80% gamma globulin by weight of total protein in the concentrate.

14. The method of claim 1 wherein said gamma globulin concentrate is a solution of Cohn Fraction II.

## Patentansprüche

1. Verfahren zur Herstellung von gamma-Globulin mit verringerter Antikomplementaktivität, das die Stufen umfaßt:
a) Bereitstellen eines gamma-Globulinkonzentrats, wobei dieses Konzentrat einen Polyether enthält und dieser Polyether in einer Konzentration vorliegt, die nicht ausreicht, um in dem Konzentrat vorhandenes Protein auszufällen, und die ausreicht, um die Antikomplementaktivität des herzustellenden gamma-Globulins zu verringern,
b) Ultrafiltration des Konzentrats, und
c) Sammeln des gamma-Globulins.

2. Verfahren nach Anspruch 1, ferner umfassend, nach Stufe b), die Stufe, daß das Konzentrat mit einem Anionenaustauscherharz behandelt wird.

3. Verfahren nach Anspruch 1, worin der Polyether im wesentlichen frei von geladenen Gruppen ist.

4. Verfahren nach Anspruch 1, worin der Polyether ein Molekulargewicht über 1000 Dalton hat.

5. Verfahren nach Anspruch 1, worin der Polyether Polyethylenglykol ist.

6. Verfahren nach Anspruch 5, worin das Polyethylenglykol ein mittleres Molekulargewicht von 4000 Dalton hat.

7. Verfahren nach Anspruch 5 oder 6, worin das Polyethylenglykol in einer Konzentration von 0,05 bis 2,0 % Gewicht/Volumen Konzentratlösung vorliegt.

8. Verfahren nach Anspruch 1, worin die Ultrafiltration bei einem pH von 5,0 bis 5,6 durchgeführt wird.

9. Verfahren nach Anspruch 1, worin die Ultrafiltration eine Dialyse des gamma-Globulinkonzentrats gegen mindestens vier Volumenteile Dialyselösung umfaßt.

10. Verfahren nach Anspruch 9, worin die Dialyselösung ebenfalls einen Polyether enthält.

11. Verfahren nach Anspruch 1, ferner umfassend das Filtrieren des gamma-Globulins, um zelluläre Mikroorganismen zurückzuhalten, Abfüllen des gamma-Globulins in Behälter, Lyophilisieren des gamma-Globulins und dichtes Verschließen der Behälter.

12. Verfahren nach Anspruch 1, worin das gamma-Globulinkonzentrat ein gamma-Globulin enthaltendes Alkoholproteinpräzipitat aus menschlichem Plasma umfaßt.

13. Verfahren nach Anspruch 1, worin das gamma-Globulinkonzentrat mehr als 80 % gamma-Globulin, bezogen auf das Gewicht des Gesamtproteins im Konzentrat, enthält.

14. Verfahren nach Anspruch 1, worin das gamma-Globulinkonzentrat eine Lösung von Cohn-Fraktion-II ist.

## Revendications

1. Procédé de préparation de gamma-globuline ayant une activité anti-complément réduite, ledit procédé comprenant les étapes de
a) mise en oeuvre d'un concentré de gamma-globuline, ledit concentré comportant un polyéther, ledit polyéther étant présent à une concentration insuffisante pour précipiter les protéines présentes dans le concentré et suffisante pour réduire l'activité anti-complément de la gamma-globuline à préparer,
b) ultrafiltration dudit concentré, et
c) recueil de ladite gamma-globuline.

2. Procédé suivant la revendication 1, comprenant en outre, après l'étape b), le traitement dudit concentré avec une résine échangeuse d'anions.

3. Procédé suivant la revendication 1, dans lequel le polyéther est essentiellement dépourvu de groupes chargés.

4. Procédé suivant la revendication 1, dans lequel le polyéther a un poids moléculaire supérieur à 1000 daltons.

5. Procédé suivant la revendication 1, dans lequel ledit polyéther est le polyéthylèneglycol.

6. Procédé suivant la revendication 5, dans lequel ledit polyéthylèneglycol a un poids moléculaire moyen de 4000 daltons.

7. Procédé suivant la revendication 5 ou 6, le polyéthylèneglycol est présent selon une concentration de 0,05 % à 2,0 % en poids par volume de solution de concentré.

8. Procédé suivant la revendication 1, dans lequel l'ultrafiltration est réalisée à un pH de 5,0 à 5,6.

9. Procédé suivant la revendication 1, dans lequel l'ultrafiltration comprend la dialyse dudit concentré de gamma-globuline contre au moins quatre volumes de la solution de dialyse.

10. Procédé suivant la revendication 9, dans lequel la solution de dialyse contient également un polyéther.

11. Procédé suivant la revendication 1, comprenant en outre la filtration de ladite gamma-globuline pour retenir les microorganismes cellulaires, le chargement de ladite gamma-globuline dans des flacons, la lyophilisation de ladite gamma-globuline et le scellement hermétique des flacons.

12. Procédé suivant la revendication 1, dans lequel ledit concentré de gamma-globuline comprend un précipité alcoolique de protéine provenant de plasma humain et contenant de la gamma-globuline.

13. Procédé suivant la revendication 1, dans lequel ledit concentré de gamma-globuline contient plus de 80 % de gamma-globuline en poids de protéine totale dans le concentré.

14. Procédé suivant la revendication 1, dans lequel ledit concentré de gamma-globuline est une solution de la Fraction II de Cohn.
